# EUROPEAN PATENT APPLICATION

(11) **EP 1 208 860 A1**
(43) Date of publication of application: **29.05.2002**
(21) Application number: 01203333.8
(22) Date of filing: 04.09.2001
(51) Int. Cl.: A61M 5/32

(54) **Protection device for a syringe for medical use**

(30) Priority: 21.11.2000 IT RE000118
(71) Applicant: C.G.M. S.P.A., 42015 Correggio (Reggio Emilia) (IT)
(72) Inventor: Parmigiani, Corrado, 42015 Corregio (Reggio Emilia) (IT); Parmigiani, Corrado Saverio, 42015 Corregio (Reggio Emilia) (IT)
(74) Representative: Corradini, Corrado

(57) **Abstract**

The device is applied to a syringe for medical use, comprising a cylindrical body, on the front end portion (12) of which there is rigidly applied a hollow needle (15) having a pointed tip (17) for penetration into the patient's body. A protection member (20) is provided, to assume alternately an open configuration and a closed configuration, to enclose, when in its closed configuration, at least the pointed tip (17) of the needle; a securing means (50) is also provided separate from the syringe (10) and able to be rigidly secured to the front end portion (12) of the syringe. Said securing means (50) is joined to the protection member (20) by connection means (55) which enable the protection member (20) to be rotated such that this can be disposed relatively far from the pointed tip (17) of the needle.

## Description

This invention relates to a needle protection device in a syringe for medical use, the syringe comprising a cylindrical body, on the front end portion of which there is rigidly applied an internally hollow needle having a pointed tip for penetration into the patient's body.

In using these needles the operator (doctor, nurse, etc.) must be protected from pricking by the needle, especially after it has been inserted into and then withdrawn from the patient's body, as it then represents a dangerous vehicle for disease transmission.

There therefore exists the problem of generally protecting those who handle syringes and in particular those who habitually operate with them from pricking by their needle, coupled with the problem of adequately and safely disposing of needles as refuse after use.

For this purpose a device was invented and patented by the same applicant (with European patent application No. 692271) presenting a front plate-like element foldable on itself to enclose the point of the needle and sufficiently rigid to prevent a person being able to be pricked by the point of the needle enclosed within it when the device has been closed.

This plate-like front element is constructed of synthetic resin and is perpetually secured to the needle by a foldable rear elongation integral with the rear part carrying the needle tube and serving as a connector to the front nosepiece of the syringe.

The element which protects the point of the needle is rigidly secured to the needle itself, and consequently during manufacture the needle shaft (of steel) has necessarily to be joined in a fixed and irremovable manner to the protection element (of plastic). As the market requires various types of shaft (differing in make, material or dimensions), the protection device has to be manufactured in a range of models, one for each type of shaft required, with consequent supply problems, generally high manufacturing costs, and stocking problems for the user hospital.

An object of the present invention is therefore to overcome said problems.

This and further objects are attained by the device of the claims.

By virtue of the invention, the protection device against needle pricking is totally free of the needle (and in particular of the shaft) and is manufactured independently thereof. Before use, the protection device is applied to the syringe, whatever the type of needle (make, model) applied to it. A single model of the protection device is therefore suitable for every type of needle.

The invention is described in detail hereinafter with the aid of the accompanying drawings, which illustrate one embodiment thereof by way of non-limiting example.

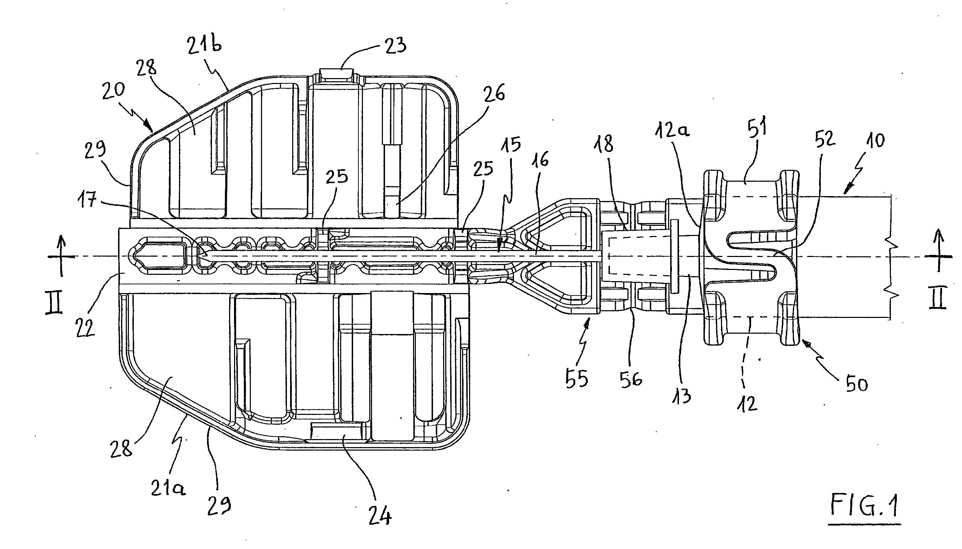

Figure 1 is a top plan view of a first embodiment of the invention.
Figure 2 is a section on the plane II-II of Figure 1.
Figure 3 is a front view in the direction of the arrow III of Figure 1, the protection member being in its open configuration.
Figure 3A is the same front view as Figure 3, but showing the protection member in its closed configuration.

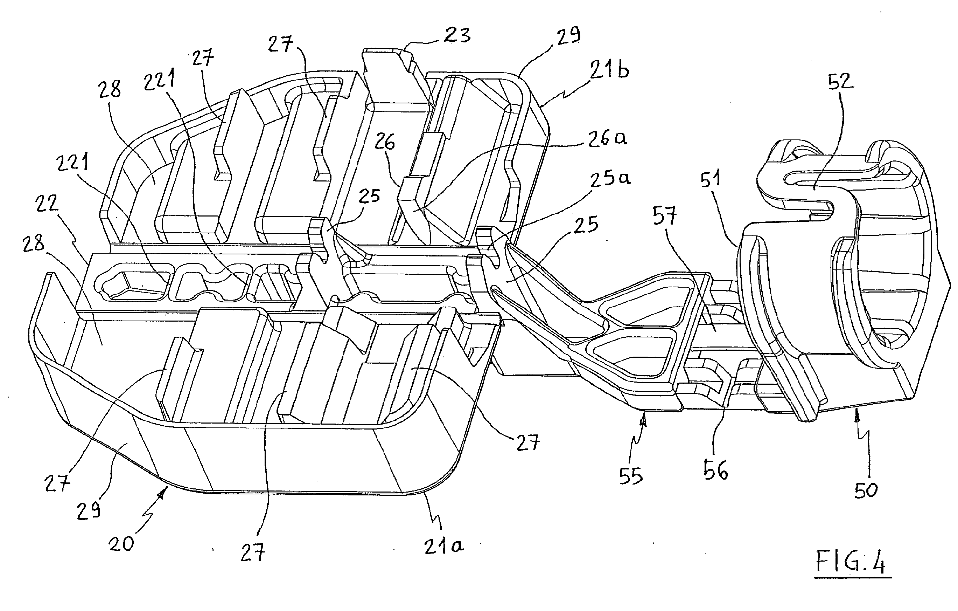

Figure 4 is a perspective view of the first embodiment of the invention.

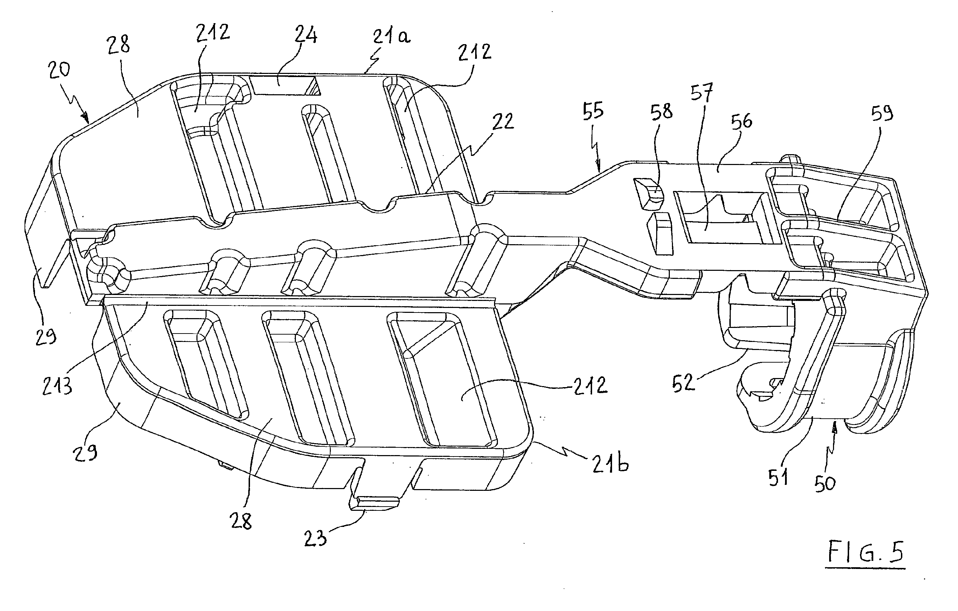

Figure 5 is another perspective view, from another viewpoint, of the device of Figure 4.

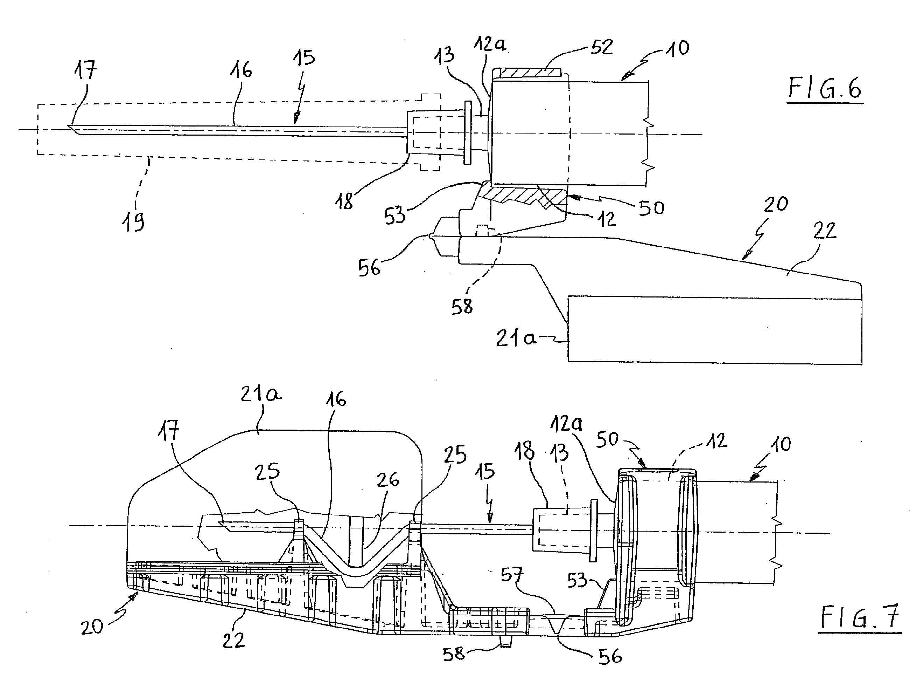

Figure 6 is a vertical side elevation of the first embodiment, with the protection member folded into a position relatively distant from the point of the needle.
Figure 7 is a side view of Figure 6, showing the protection member in its closed configuration about the needle.
Figure 8 is a top plan view of a second embodiment of the invention.
Figure 9 is a front view in the direction of the arrow IX of Figure 8, with the protection member in its open configuration.
Figure 9A is the same view as Figure 9, with the protection member in its closed configuration.

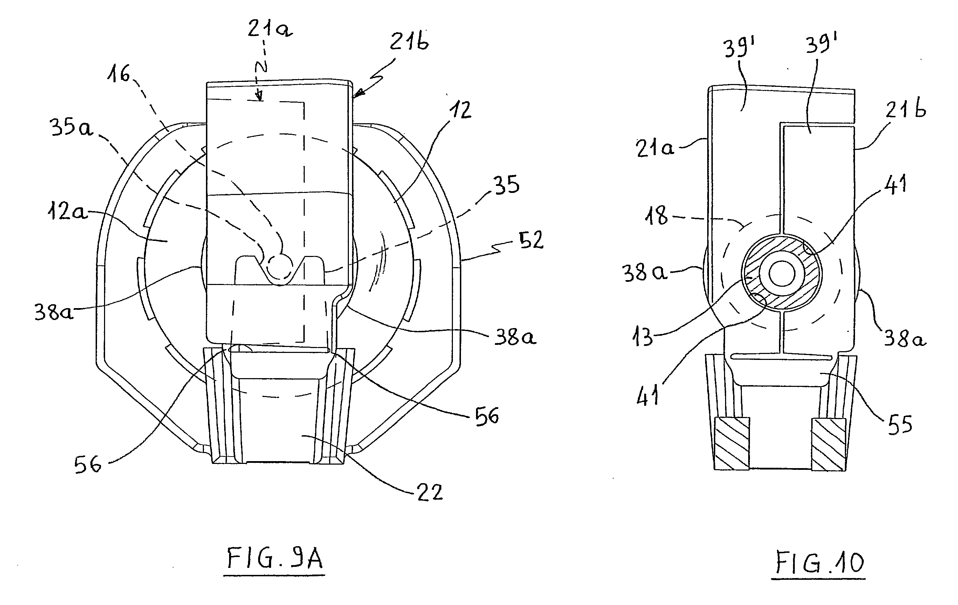

Figure 10 is a section on the plane X-X of Figure 8, with the protection member in its closed configuration.

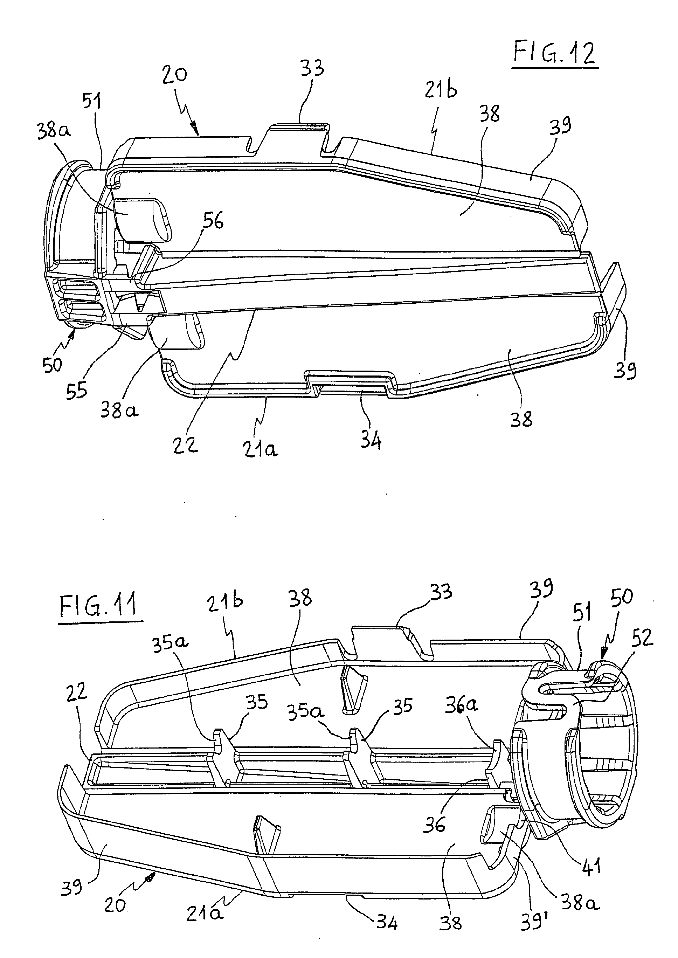

Figure 11 is a perspective view of the second embodiment.
Figure 12 is another perspective view, from another viewpoint, of the device of Figure 11.

The device of the invention is intended to be applied to a syringe 10 for medical use, having a cylindrical body, on the front end portion of which there is applied a needle 15 having a hollow shaft 16 provided with a pointed tip 17 for penetration into the patient's body.

Specifically, the needle illustrated in the figures is of the most usual type in which the shaft 16 is joined at its rear end to a connector piece 18 ("Luer" connector), to receive by axial insertion a conduit 13 (nosepiece) projecting from the front end portion 12 of the cylindrical body and communicating with the internal cavity of the syringe.

The device of the invention comprises a protection member 20 arranged to assume an open configuration and a closed configuration to enclose, when in its closed configuration, at least a part of the needle 15, namely that comprising the pointed tip 17.

The protection member 20 is associated with the syringe 10 by a securing means 50 separate from the syringe 10, to be securely fixed to the front end portion 12 of the cylindrical body and connected to the protection member 20 by connection means which enable the protection member 20 to rotate so that it can be disposed relatively far from the pointed tip 17 of the needle.

In a preferred (but not exclusive) embodiment shown in the figures, said securing means 50 comprises an annular part 51 having a cylindrical hole enabling it to insertingly embrace the front end portion 12 of the cylindrical body with a force sufficient to rigidly secure them together.

In particular, said annular part 51 is in the form of a relatively thin annular band of synthetic resin with an elastically expandable circumference and is in geometrical relationship with said front end portion 12 such as to embrace this with an elastic reaction sufficient to rigidly secure them together. Said elastic reaction is provided by a thinner ring portion 52 in the form of a broken line which defines an arc having a length which is elastically extensible. By virtue of the elastic portion 52, the annular part 51 is expandable to the point of being able to be applied to front end portions 12 having diameters which differ by at least 5 mm. When the annular part 51 has been forced onto the front end portion 12, the elastic reaction provided by the portion 52 forcibly secures the part 51 to the syringe.

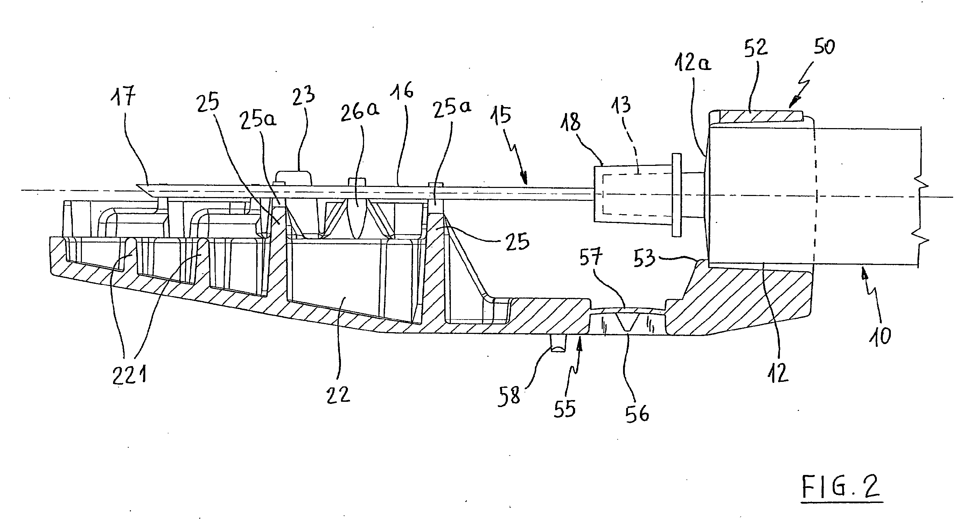

A shoulder 53 acting as a stop against the forward front face 12a (see Figure 2 in particular) enables the part 51 to be positioned on the front end portion 12 in a constant and precise geometrical relationship with the front face 12a.

The protection member 20 is formed of synthetic resin and comprises a relatively thin central portion 22 which is elongate parallel to the needle 15, is stiffened by inner ribs 221 and is joined to the connection means by the securing means 50, and two movable half-shells 21a, 21b, connected to the portion 22 in a manner enabling them to rotate.

The two half-shells 21a, 21b are connected together by the central portion 22 and can be moved towards each other such that when in the closed configuration they face each other and are partially inserted one into the other to define a closed chamber which encloses at least a part of the needle 15, namely that comprising the pointed tip 17.

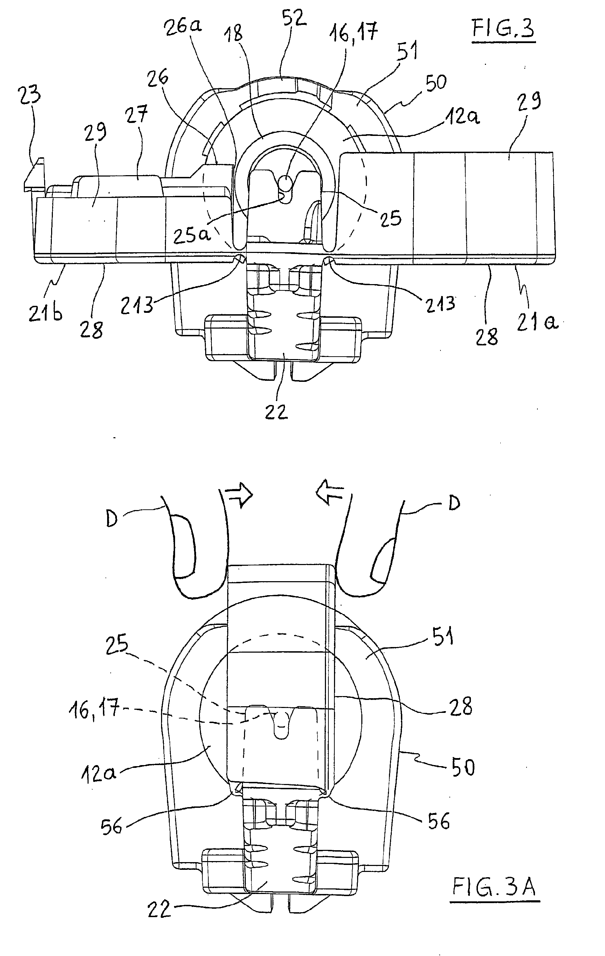

The two half-shells 21a, 21b are box-like, one (21b) of the two partially penetrating into the other when they are brought together. In detail, they are each composed of an overall flat major surface 28 which is parallel to the other surface 28 when they are in their closed configuration (see Figure 3A in particular), and a peripheral wall 29 perpendicular to the surface 28 and extending along three sides of the edge of the surface 28, to leave the side adjacent to the central portion 22 free. The major surfaces 28 are stiffened by each comprising a number (three in the figures) of concavities 212.

The major surfaces 28 are joined to the sides of the central portion 22, the joining line 213 being thin to enable the surfaces 28 to be rotated about the portion 22. The protection member 20 comprises snap-connection means to fix the half-shells 21a, 21b together when brought into their closed configuration. For example, a coupling tooth 23 can be provided on the upper edge of one of the walls 29, to be snap-inserted into and coupled to a slot 24 provided in the wall 29 of the opposite half-shell, to lock the two mutually facing half-shells together.

In the illustrated embodiment, pressing means are provided to press against the shaft 16 of the needle at points axially spaced apart, to deform the needle as a result of moving the half-shells 21a, 21b towards each other.

Specifically, first pressing elements 25 in the form of projections projecting upwards from the central portion 22 are provided, having in their upper end forkshaped seats 25a to restingly receive and embrace the shaft 16 of the needle when the protection member 20 is brought alongside the needle 15 (as shown in Figure 2). At least one second pressing element 26 carried by the half-shells 21a, 21b is also provided, consisting of a transverse rib perpendicular to the respective major surface 28 and having its inner side positioned substantially vertical in proximity to the central portion 22 to the side of the seats 25a when the half-shells 21a, 21b are in their open configuration (see Figure 3 in particular) and coplanar.

Said element 26 is arranged to come into contact with the needle shaft 16 in a point axially spaced from the seats 25a when the two half-shells 21a, 21b are brought together, and to deform it permanently when the half-shells are closest together (closed configuration).

In the illustrated embodiment only one second pressing element 26 is provided, positioned on one of the two half-shells 21a, 21b. Three ribs 27 parallel to the ribs 26 are also provided to stiffen the half-shells 21a, 21b, however their inner sides do not interfere with the shaft 16.

The entire protection device, i.e. the protection member 20, the securing means and the means for connecting them together, are formed of synthetic resin in one piece; said connection means are defined by a longitudinal elongate element 55, which joins the central portion 22 to the annular part 51 by bridging, and has at least one folding region 56 which, in relation to the elasticity of the material, is sufficiently thin to enable the protection member 20 to rotate relative to the securing means 50.

In the initial configuration of the device, prior to its use, the two half-shells 21a, 21b are in their open configuration, and straddling the folding region 56 there is provided a tang 57 which joins together the two parts of the element 55 lying to the right and to the left of the region 56, and maintains the element 55 in a rectilinear (non-folded) configuration; in this configuration, the protection member 20 lies relatively far from the securing means 50.

To enable the protection member 20 to rotate such that it becomes disposed relatively far from the point 17 of the needle, the tang 57 has to be broken, this requiring only a relatively small force.

The device of the invention is applied to the syringe 10 before this is used, by mounting the annular part 51 about the front end portion 12 so that the shoulder 53 is brought into contact with the edge of the front face 12a of the syringe. This operation can be carried out easily and without danger by using the syringe without the needle applied, in which case the needle 15 can be applied to the syringe afterwards, preferably together with its protection cap. Alternatively it can be carried out on the syringe to which the needle has already been applied, checking however that the needle is protected by the cap 19.

The protection member 20 is then rotated rearwards through about 180 degrees (tearing the tang 57 by this operation) and brought to the side of the cylindrical body of the syringe and hence distant from the needle 15, which is then completely free to penetrate into the patient's body. In this configuration, the syringe and its needle can be used in the typical traditional manner on the patient's body, as the protection member 20 is of no hindrance. This configuration is maintained stable by the fact that a fork 58 projecting downwards from the lower surface of the element 55, at a point to the front of the folding region 56, forcibly receives by insertion a rib 59 located on the lower part of the annular member 51, and hence secures to the annular part 15 that part of the element 55 lying to the front of the region 56.

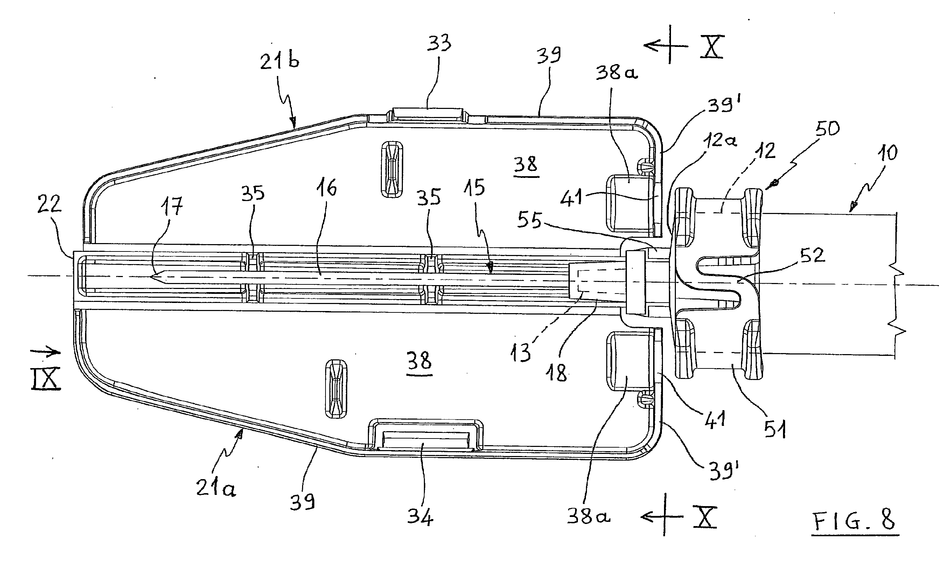

After use, to prevent any danger of pricking by the point 17, the protection member 20, in its open configuration, is again rotated by turning it with the hand into the preceding position, and brought alongside the shaft 16. In this position, the shaft is inserted into the two seats 25a. At this point, the two half-shells 21a, 21b are rotated towards each other by pushing with two fingers D of the same hand against the most spaced apart ends of the half-shells (Figure 3A). As a result of this movement, the inner side 26a of the pressing element 26 interferes with the geometrical axis of the needle 15, hence by pressing against the shaft 16 while this is resting in the seats 25a, it substantially deforms its axis permanently (Figure 7). At the end of their closure movement, the two half-shells 21a, 21b are locked together by the tooth 23 which snap-inserts into the slot 24; the point 17 of the needle remains closed within the protection member 20, and by virtue of the elastic reaction of the deformed shaft 16 against the respective pressing elements 25 and 26 the needle 15 remains secured to these, their withdrawal from the member 20 being prevented. At this point, the needle 15 is confined and enclosed within the member 20 and can be removed from the syringe and disposed of without any danger of pricking, or can be thrown away together with the syringe.

The second illustrated embodiment is applied to the case in which the needle comprises a hollow shaft 16 joined at its rear end to an axial connector piece for mounting on a conduit 13 projecting from the front end portion 12 of the cylindrical body of the sylinge.

This embodiment differs from the first mainly in that it does not comprise a second pressing element 26, the needle geometry not being deformed by closure of the half-shells 21a, 21b of the protection member 20.

Instead, this embodiment is characterised in that the protection member 20 is of such dimensions and in such geometrical relationship with the needle 15 that it encloses within its interior the entire needle 15, from its point 17 to its connector piece 18, when the two half-shells 21a, 21b are closed.

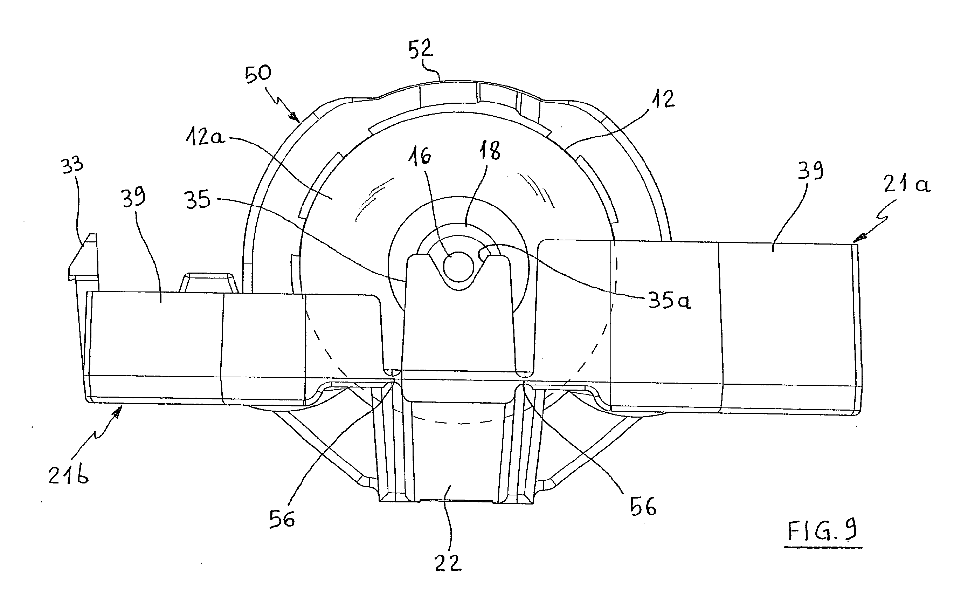

In detail, the half-shells 21a, 21b are box-like and are each composed of a horizontal, substantially flat major surface 38 which is coplanar with the other surface 38 when they are in their open configuration (see Figure 9 in particular), and a peripheral wall 39 perpendicular to the surface 38 and extending along three sides of the edge of the surface 38, to leave the side free at the central portion 22.

The rear portion of the half-shells 21a, 21b is shaped to house virtually as an exact fit the needle connector piece 18 when the member 20 is in its closed configuration. In particular, on the rear portion of the major surfaces 38 there is provided a convex region 38a which defines a concave seat to receive the piece 18 as an exact fit. In addition, in the rear side 39' of each wall 39 there is provided an aperture 41 which embraces and receives the conduit 13 of the syringe as an exact fit and is inserted to the rear of the connector piece 18 and to the front of the face 12a.

Snap-coupling means are again provided to lock together the half-shells 21a, 21b when these are brought into their closed configuration. For example, a coupling tooth 33 can be provided on the upper edge of one of the walls 39, to be snap-inserted into and coupled to a slot 34 provided in the wall 39 of the opposite half-shell, to lock the two mutually facing half-shells together.

The longitudinal dimension of the half-shells 21a, 21b is greater than the total length of the needle 15, the geometrical position which the protection member 20 assumes relative to the syringe 10 being such that when closed, it forms a closed chamber which encloses in its interior the entire needle 15.

In a like manner to the first embodiment, pressing elements 35, 36 are provided in the form of vertical projections projecting upwards from the central portion 22, having in their upper end fork-shaped seats 35a, 36a to restingly receive and embrace the shaft 16 of the needle and the piece 18 when the protection member 20 is brought alongside the needle 15.

In use, this second embodiment is applied to the syringe 10, the protection member 20 is then rotated rearwards to the side of the syringe, and the syringe and its needle 15 are used in the typical traditional manner, after which the protection member 20 is closed about the needle 15, all as in the case of the first embodiment. The difference lies in the fact that the shaft 16 is not deformed on closing the member 20, however the entire needle 15 remains confined within the chamber defined in the interior of the member 20, the presence of the rear side 39' of the half-shells, which embraces the conduit 13 by a hole (formed from the apertures 41) of diameter less than the connector piece 18, ensuring that the needle 15 cannot escape through said hole.

Numerous modifications of a practical and applicational nature can be made to the invention, but without deviating from the scope of the inventive idea as claimed below.

## Claims

1. A needle protection device in a sylinge for medical use, comprising a cylindrical body, on the front end portion (12) of which there is rigidly applied a hollow needle (15) having a pointed tip (17) for penetration into the patient's body,
**characterised by** comprising:
a protection member (20) arranged to assume alternately an open configuration and a closed configuration, to enclose, when in its closed configuration, at least the pointed tip (17) of the needle; and
a securing means (50), separate from the syringe (10) and able to be rigidly secured to the front end portion (12) of the syringe;
said securing means (50) being joined to the protection member (20) by connection means (55) which enable the protection member (20) to be rotated such that this can be disposed relatively far from the pointed tip (17) of the needle.

2. A device as claimed in claim 1, **characterised in that** said securing means (50) comprise an annular part (51) able to insertingly embrace said front end portion (12) of the syringe with a force sufficient to rigidly secure them together.

3. A device as claimed in claim 2, **characterised in that** said annular part (51) has an elastically expandable circumference, and is in geometrical relationship with said front end portion (12) such as to embrace this with an elastic reaction sufficient to rigidly secure them together.

4. A device as claimed in claim.3, **characterised in that** said annular part (51) is expandable to the point of being able to be applied to front end portions (12) having diameters which differ by at least 5 mm.

5. A device as claimed in claim 3, **characterised in that** said annular part (51) is of synthetic resin and possesses a portion (52) in the form of a broken line which defines an arc having a length which is elastically extensible.

6. A device as claimed in claim 2, **characterised by** being formed in one piece from synthetic resin, the means for connecting the protection member (20) to the securing means (50) being defined by a longitudinal elongate element (55) which joins the central portion (22) of the protection member (20) to said annular part (51), and has at least one folding region (56) which, in relation to the elasticity of the material, is sufficiently thin to enable the protection member (20) to rotate relative to the securing means (50).

7. A device as claimed in claim 1, **characterised in that** the protection member (20) comprises two half-shells (21a, 21b) connected together and movable towards each other such that when in said closed configuration they face each other and are partially inserted one into the other to define a closed chamber which encloses at least that part of the needle 15 comprising the pointed tip (17).

8. A device as claimed in claim 1, **characterised in that** the protection member (20) comprises means (23, 24) for locking said half-shells (21a, 21b) together when these are brought into their closed configuration, and pressing elements (25, 26) arranged to press against the shaft (16) of the needle at points axially spaced apart, to deform the needle as a result of moving the half-shells (21a, 21b) towards each other.

9. A device as claimed in claim 8, **characterised in that** the protection member (20) comprises, joined to said connection means (55), a relatively narrow, elongate central portion (22) to which said movable half-shells (21a, 21b) are secured in a manner enabling them to rotate.

10. A device as claimed in claim 9, **characterised by** comprising first pressing elements (25) carried by the central portion (22) and arranged to restingly receive the shaft (16) of the needle when the protection member (20) is brought alongside the needle (15), and second pressing elements (26) carried by the half-shells (21a, 21b), to come into contact with the needle (15) at points axially spaced apart when the two half-shells (21a, 21b) are brought together.

11. A device as claimed in claim 7, said needle comprising a hollow shaft (16) joined at its rear end to a connector piece (18) for axial insertion by a conduit (13) projecting from the portion (12) of the syringe, **characterised in that** the protection member (20) is in such geometrical relationship with the needle (15) and is of such dimensions that it encloses within its interior the entire needle (15) when the two half-shells (21a, 21b) are brought into their closed configuration.

12. A device as claimed in claim 11, **characterised in that** each half-shell (21a, 21b) comprises a rear side (39') having an aperture (41) which, when the member (20) is in its closed configuration, embraces and receives as an exact fit the conduit (13) of the syringe and is inserted to the rear of the connector piece (18) and to the front of the face (12a).

13. A device as claimed in claim 11, **characterised in that** the rear portion of the half-shells (21a, 21b) is shaped to house the connector piece (18) of the needle virtually as an exact fit, when the member (20) is in its closed configuration.
